# EUROPÄISCHE PATENTSCHRIFT

(11) **EP 3 441 383 B1**
(45) Veröffentlichungstag und Bekanntmachung des Hinweises auf die Patenterteilung: **25.12.2019**
(21) Anmeldenummer: 17185346.8
(22) Anmeldetag: 08.08.2017
(51) Int. Cl.: C07C 67/38, C01B 32/39, C07C 69/00, C07C 69/02

(54) **METHOXYCARBONYLIERUNG MIT AMEISENSÄURE ALS CO-QUELLE**
METHOXY CARBONYLATION WITH FORMIC ACID AS CO SOURCE
MÉTHOXYCARBONYLATION AU MOYEN D'ACIDE FORMIQUE COMME SOURCE DE CO

(43) Veröffentlichungstag der Anmeldung: 13.02.2019
(73) Patentinhaber: Evonik Operations GmbH, 45128 Essen (DE)
(72) Erfinder: SANG, Rui, 252000 Liaocheng City Shandong Province (CN); LIU, Jie, 17172 Solna (SE); DONG, Kaiwu, 236800 Bo Zhou (CN); JACKSTELL, Ralf, 10106 Rostock (DE); BELLER, Matthias, 18211 Ostseebad (DE); FRANKE, Robert, 45772 Marl (DE)
(74) Vertreter: Evonik Patent Association

(56) Entgegenhaltungen:
- EP-A2- 3 121 185
- KAIWU DONG ET AL: "Palladium-Catalyzed Carbonylation of sec - and tert -Alcohols", ANGEWANDTE CHEMIE INTERNATIONAL EDITION, Bd. 56, Nr. 22, 21. April 2017 (2017-04-21), Seiten 6203-6207, XP55426870, ISSN: 1433-7851, DOI: 10.1002/anie.201701950
- TSUMORU MORIMOTO ET AL: "Evolution of carbonylation catalysis: no need for carbon monoxide", ANGEWANDTE CHEMIE INTERNATIONAL EDITION, WILEY-VCH VERLAG GMBH , Bd. 43, Nr. 42 25. Oktober 2004 (2004-10-25), Seiten 5580-5588, XP002668525, ISSN: 1433-7851, DOI: 10.1002/ANIE.200301736 Gefunden im Internet: URL:http://onlinelibrary.wiley.com/doi/10. 1002/anie.200301736/abstract [gefunden am 2004-09-01]

## Beschreibung

Die Erfindung betrifft ein Verfahren zur Methoxycarbonylierung mit Ameisensäure als CO-Quelle.

Die Methoxycarbonylierung von Alkenen ist ein Prozess mit steigender Bedeutung. Bei der klassischen Methoxycarbonylierung wird ein Olefin mit CO und MeOH in Gegenwart eines Katalysators, welcher einen Liganden und ein Metall umfasst, umgesetzt:

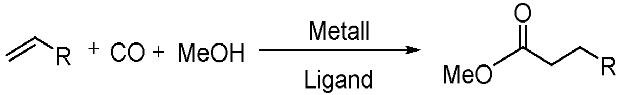

Hierbei wird CO als Gas in das Reaktionsgefäß eingeleitet (z.B. Angew. Chem. Int. Ed. 2017, 56, 6203).

Der Erfindung lag die Aufgabe zugrunde, ein Verfahren bereitzustellen, bei welchem eine andere CO-Quelle zum Einsatz kommt als CO-Gas, welches in das Reaktionsgefäß eingeleitet wird. Bei dem Verfahren sollte eine gute Ausbeute an Methylester erzielt werden.

Gelöst wird die Aufgabe durch ein Verfahren gemäß Anspruch 1.

Verfahren umfassend die Verfahrensschritte:
a) Zugabe eines Olefins;
b) Zugabe einer Verbindung, welche Pd umfasst, wobei das Pd in der Lage ist einen Komplex auszubilden;
c) Zugabe einer Verbindung der allgemeinen Formel (**I**): 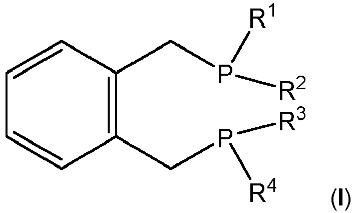
   wobei R¹, R², R³, R⁴ jeweils unabhängig voneinander ausgewählt sind aus: -H, -(C₁-C₁₂)-Alkyl, - O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
   wobei die genannten Alkylgruppen, Arylgruppen, Cycloalkyl, Heteroalkylgruppen, Heteroarylgruppen wie folgt substituiert sein können:
      -(C₁-C₁₂)-Alkyl, -O-(C₁-C₁₂)-Alkyl, Halogen;
   und mindestens einer der Reste R¹, R², R³, R⁴ nicht für Phenyl steht;
d) Zugabe von MeOH und HCOOH,
   wobei das Verhältnis MeOH / HCOOH bezogen auf das eingesetzte Volumen im Bereich von 1,55 : 0,45 bis 1,1 : 0,9 liegt;
e) Erhitzen des Reaktionsgemischen, wobei das Olefin zum Methylester umgesetzt wird.

In einer Variante des Verfahrens wird dem Reaktionsgemisch kein CO-Gas zugeführt.

In einer Variante des Verfahrens dient HCOOH als einzige CO-Quelle für die Reaktion.

In einer Variante des Verfahrens ist die Verbindung in Verfahrensschritt b) ausgewählt aus: Pd(acac)₂, PdCl₂, Pd(dba)₃*CH₃Cl (dba = Dibenzylidenaceton), Pd(OAc)₂, Pd(TFA)₂, Pd(CH₃CN)Cl₂.

In einer Variante des Verfahrens ist die Verbindung in Verfahrensschritt b) Pd(OAc)₂.

In einer Variante des Verfahrens umfasst das Verfahren den zusätzlichen Verfahrensschritt f):
f) Zugabe einer Säure.

In einer Variante des Verfahrens ist die Säure ausgewählt aus: H₂SO₄, CH₃SO₃H, CF₃SO₃H, PTSA (p-Toluolsulfonsäure).

In einer Variante des Verfahrens ist die Säure PTSA (p-Toluolsulfonsäure).

In einer Variante des Verfahrens liegt das Verhältnis MeOH / HCOOH bezogen auf das eingesetzte Volumen im Bereich von 1,5 : 0,5 bis 1,2 : 0,8.

In einer Variante des Verfahrens sind R¹, R², R³, R⁴ jeweils unabhängig voneinander ausgewählt aus: -(C₁-C₁₂)-Alkyl, -O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, - COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

In einer Variante des Verfahrens sind R¹, R², R³, R⁴ jeweils unabhängig voneinander ausgewählt aus: -(C₁-C₁₂)-Alkyl, -(C₄-C₁₄)-Aryl, Cycloalkyl, -(C₁-C₁₂)-Heteroalkyl, -(C₃-C₁₄)-Heteroaryl, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

In einer Variante des Verfahrens sind R¹, R², R³, R⁴ jeweils unabhängig voneinander ausgewählt aus: -(C₁-C₁₂)-Alkyl, Cycloalkyl, -(C₃-C₁₄)-Heteroaryl und die Reste auch zur Bildung eines größeren kondensierten Ringes befähigt sind;
wobei die genannten Alkylgruppen, Cycloalkyl, Heteroarylgruppen wie folgt substituiert sein können:
-(C₁-C₁₂)-Alkyl, -O-(C₁-C₁₂)-Alkyl, Halogen,
und mindestens einer der Reste R¹, R², R³, R⁴ nicht für Phenyl steht.

In einer Variante des Verfahrens sind R¹, R⁴ jeweils unabhängig voneinander ausgewählt aus: - (C₁-C₁₂)-Alkyl, Cycloalkyl und die Reste auch zur Bildung eines größeren kondensierten Ringes befähigt sind;
wobei die genannten Alkylgruppen, Cycloalkyl wie folgt substituiert sein können:
-(C₁-C₁₂)-Alkyl, -O-(C₁-C₁₂)-Alkyl, Halogen.

In einer Variante des Verfahrens stehen R², R³ jeweils unabhängig voneinander für -(C₃-C₁₄)-Heteroaryl,
wobei die genannten Heteroarylgruppen wie folgt substituiert sein können:
-(C₁-C₁₂)-Alkyl, -O-(C₁-C₁₂)-Alkyl, Halogen.

In einer Variante des Verfahrens weist die Verbindung der allgemeinen Formel (**I**) die Struktur (2) auf:

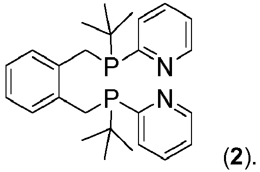

In einer Variante des Verfahrens weist die Verbindung der allgemeinen Formel (**I**) die Struktur (3) auf:

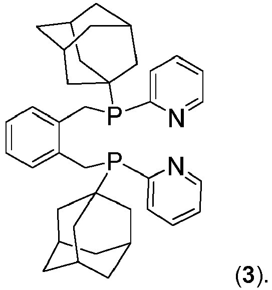

Neben dem Verfahren wird auch eine Verbindung an sich beansprucht.

Verbindung, welche die Struktur (3) aufweist:

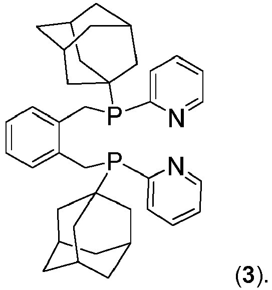

Im Folgenden wird die Erfindung anhand von Ausführungsbeispielen näher erläutert.

### A) Pd-katalysierte Methoxycarbonylierung von Tetramethylethylen 1a mit HCOOH: Einfluss des Verhältnisses von MeOH zu HCOOH

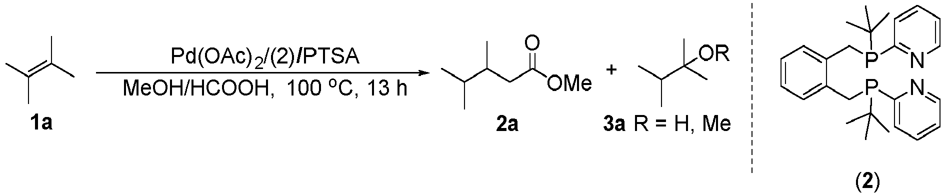

In ein verschlossenes 35-ml-Röhrchen wurden [Pd(OAc)₂] (1,12 mg, 0,25 Mol-%), (**2**) (8,72 mg, 1,0 Mol-%), p-Toluolsulfonsäure (PTSA·H₂O) (15,2 mg, 4 Mol-%) und ein ofengetrocknetes Rührstäbchen gegeben. Das Röhrchen wurde zusammen mit dem Deckel in ein langes Schlenk-Rohr mit großer Öffnung gegeben. Das Schlenk-Rohrwird dreimal evakuiert und wieder mit Argon aufgefüllt. Unter einer Argonatmosphäre wurden **1a** (2 mmol), MeOH (X ml) und HCOOH (Y ml) (X ml + Y ml = 2 ml) mittels einer Spritze in das 35-ml-Röhrchen injiziert. Das 35-ml-Röhrchen wurde dann mit dem Deckel verschlossen. Die Reaktion erfolgte über 13 h bei 100 °C. Nach dem Ende der Umsetzung wurde das Röhrchen ohne zusätzliche Kühlung auf Raumtemperatur kommen gelassen und es wurde vorsichtig entspannt. Dann wurde Isooctan (100 µl) als innerer Standard injiziert. Der Umsatz wurde durch GC-Analyse gemessen.

In der folgenden Tabelle 1 sind die Ergebnisse zusammengefasst:

**Tabelle 1:**

| MeOH/HCOOH (zusammen 2 ml) | Umsatz % | **2a** Ausbeute % | **3a** Ausbeute % |
|---|---|---|---|
| 1.8/0.2 | 63 | 36 | 24 |
| 1.6/0.4 | 70 | 49 | 19 |
| 1.5/0.5 | 76 | 62 | 13 |
| 1.4/0.6 | 76 | 61 | 13 |
| 1.2/0.8 | 78 | 66 | 10 |
| 1.0/1.0 | 74 | 48 | 10 |

### B) Pd-katalysierte Methoxycarbonylierung von Tetramethylethylen 1a mit HCOOH: Einfluss des Liganden

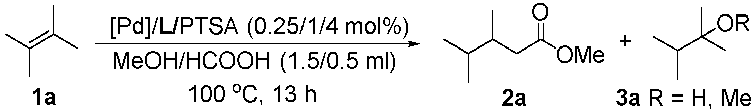

Unter einer Argonatmosphäre wurden [Pd(OAc)₂] (1,12 mg, 0,25 Mol-%), Ligand (1 Mol-%), p-Toluolsulfonsäure (PTSA·H₂O) (15,2 mg, 4 Mol-%) und ein ofengetrocknetes Rührstäbchen in ein verschlossenes 35-ml-Röhrchen gegeben. Das Röhrchen wurde zusammen mit dem Deckel in ein langes Schlenk-Rohr mit großer Öffnung gegeben. Das Schlenk-Rohr wurde dreimal evakuiert und wieder mit Argon aufgefüllt. **1a** (2 mmol), HCOOH (0,5 ml) und MeOH (1,5 ml) wurden mittels einer Spritze in das 35-ml-Röhrchen injiziert. Das 35-ml-Röhrchen wurde dann mit dem Deckel verschlossen. Die Reaktion erfolgte über 13 h bei 100 °C. Nach dem Ende der Umsetzung wurde das Röhrchen ohne zusätzliche Kühlung auf Raumtemperatur kommen gelassen (wird sehr kaltes Wasser verwendet, kann das Röhrchen bersten) und es wurde vorsichtig entspannt. Dann wurde Isooctan (100 µl) als innerer Standard injiziert. Der Umsatz wurde durch GC-Analyse gemessen.

In der folgenden Tabelle 2 sind die Ergebnisse zusammengefasst:

**Tabelle 2:**

| Ligand (L) | Umsatz % | **2a** Ausbeute % | **3a** Ausbeute % |
|---|---|---|---|
| 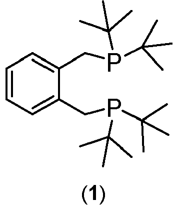 | 60 | 32 | 22 |
| 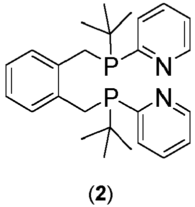 | 76 | 62 | 13 |
| 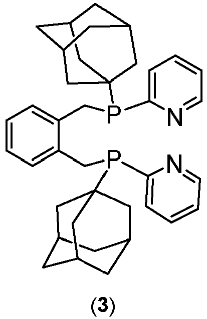 | 83 | 67 | 13 |
| 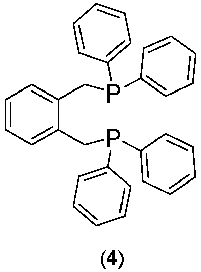 | 40 | 0 | 35 |
| 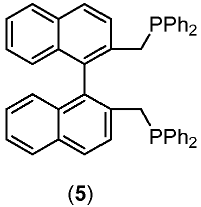 | 38 | 0 | 31 |

Wie die oben beschriebenen Versuche zeigen, wird die Aufgabe durch ein erfindungsgemäßes Verfahren gelöst.

## Patentansprüche

1. Verfahren umfassend die Verfahrensschritte:
a) Zugabe eines Olefins;
b) Zugabe einer Verbindung, welche Pd umfasst, wobei das Pd in der Lage ist einen Komplex auszubilden;
c) Zugabe einer Verbindung der allgemeinen Formel (**I**): 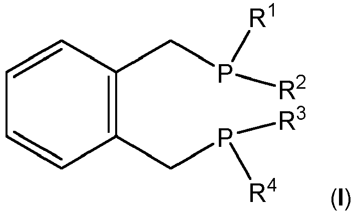
wobei R¹, R², R³, R⁴ jeweils unabhängig voneinander ausgewählt sind aus: -H, -(C₁-C₁₂)-Alkyl, - O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht;
d) Zugabe von MeOH und HCOOH,
wobei das Verhältnis MeOH / HCOOH bezogen auf das eingesetzte Volumen im Bereich von 1,55 : 0,45 bis 1,1 : 0,9 liegt;
e) Erhitzen des Reaktionsgemischen, wobei das Olefin zum Methylester umgesetzt wird.

2. Verfahren nach Anspruch 1,
wobei dem Reaktionsgemisch kein CO-Gas zugeführt wird.

3. Verfahren nach Anspruch 1 oder 2,
wobei HCOOH als einzige CO-Quelle für die Reaktion dient.

4. Verfahren nach einem der Ansprüche 1 bis 3,
wobei die Verbindung in Verfahrensschritt b) ausgewählt ist aus:
Pd(acac)₂, PdCl₂, Pd(dba)₃*CH₃Cl (dba = Dibenzylidenaceton), Pd(OAc)₂, Pd(TFA)₂, Pd(CH₃CN)Cl₂.

5. Verfahren nach einem der Ansprüche 1 bis 4,
wobei das Verfahren den zusätzlichen Verfahrensschritt f) umfasst:
f) Zugabe einer Säure.

6. Verfahren nach Anspruch 5,
wobei die Säure ausgewählt ist aus: H₂SO₄, CH₃SO₃H, CF₃SO₃H, PTSA.

7. Verfahren nach einem der Ansprüche 1 bis 6,
wobei das das Verhältnis MeOH / HCOOH bezogen auf das eingesetzte Volumen im Bereich von 1,5 : 0,5 bis 1,2 : 0,8 liegt.

8. Verfahren nach einem der Ansprüche 1 bis 7,
wobei R¹, R², R³, R⁴ jeweils unabhängig voneinander ausgewählt sind aus: -(C₁-C₁₂)-Alkyl, -O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

9. Verfahren nach einem der Ansprüche 1 bis 8,
wobei R¹, R², R³, R⁴ jeweils unabhängig voneinander ausgewählt sind aus: -(C₁-C₁₂)-Alkyl, -(C4-C₁₄)-Aryl, Cycloalkyl, -(C₁-C₁₂)-Heteroalkyl, -(C₃-C₁₄)-Heteroaryl, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

10. Verfahren nach einem der Ansprüche 1 bis 9,
wobei R¹, R², R³, R⁴ jeweils unabhängig voneinander ausgewählt sind aus: -(C₁-C₁₂)-Alkyl, Cycloalkyl, -(C₃-C₁₄)-Heteroaryl und die Reste auch zur Bildung eines größeren kondensierten Ringes befähigt sind;
wobei die genannten Alkylgruppen, Cycloalkyl, Heteroarylgruppen wie folgt substituiert sein können:
-(C₁-C₁₂)-Alkyl, -O-(C₁-C₁₂)-Alkyl, Halogen,
und mindestens einer der Reste R¹, R², R³, R⁴ nicht für Phenyl steht.

11. Verfahren nach einem der Ansprüche 1 bis 10,
wobei R¹, R⁴ jeweils unabhängig voneinander ausgewählt sind aus: -(C₁-C₁₂)-Alkyl, Cycloalkyl und die Reste auch zur Bildung eines größeren kondensierten Ringes befähigt sind;
wobei die genannten Alkylgruppen, Cycloalkyl wie folgt substituiert sein können:
-(C₁-C₁₂)-Alkyl, -O-(C₁-C₁₂)-Alkyl, Halogen.

12. Verfahren nach einem der Ansprüche 1 bis 11,
wobei R², R³ jeweils unabhängig voneinander für -(C₃-C₁₄)-Heteroaryl stehen,
wobei die genannten Heteroarylgruppen wie folgt substituiert sein können:
-(C₁-C₁₂)-Alkyl, -O-(C₁-C₁₂)-Alkyl, Halogen.

13. Verfahren nach einem der Ansprüche 1 bis 12,
wobei die Verbindung der allgemeinen Formel (**I**) die Struktur (**2**) aufweist:

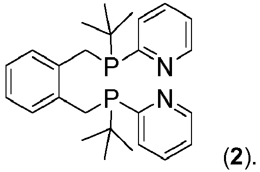

14. Verfahren nach einem der Ansprüche 1 bis 12,
wobei die Verbindung der allgemeinen Formel (**I**) die Struktur (**3**) aufweist:

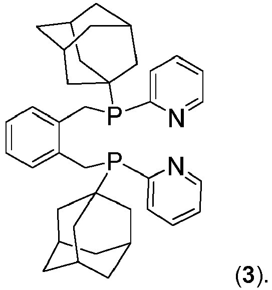

15. Verbindung, welche die Struktur (**3**) aufweist:

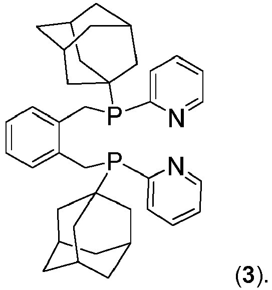

## Claims

1. Process comprising the process steps of:
a) addition of an olefin;
b) addition of a compound comprising Pd, wherein the Pd is capable of forming a complex;
c) addition of a compound of general formula (**I**): 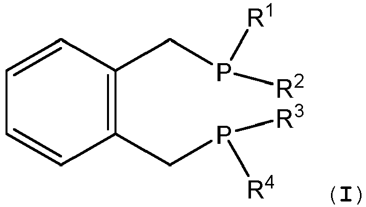
wherein R¹, R², R³, R⁴ are each independently selected from: -H, -(C₁-C₁₂) -alkyl, -O-(C₁-C₁₂) -alkyl, -(C₄-C₁₄)-aryl, -O-(C₄-C₁₄)-aryl, cycloalkyl, -(C₁-C₁₂) -heteroalkyl, -O-(C₁-C₁₂) -heteroalkyl, -(C₃-C₁₄) -heteroaryl, -O-(C₃-C₁₄) - heteroaryl, -COO-alkyl, -COO-aryl, -C-O-alkyl, -C-O-aryl, NH₂, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl;
d) addition of MeOH and HCOOH,
wherein the MeOH / HCOOH ratio based on the employed volume is in the range from 1.55 : 0.45 to 1.1 : 0.9;
e) heating the reaction mixture to convert the olefin into the methyl ester.

2. Process according to Claim 1,
wherein no CO gas is supplied to the reaction mixture.

3. Process according to Claim 1 or 2,
wherein HCOOH serves as the only CO source for the reaction.

4. Process according to any of Claims 1 to 3,
wherein the compound in process step b) is selected from:
Pd(acac)₂, PdCl₂, Pd(dba)₃*CH₃Cl (dba = dibenzylideneacetone), Pd(OAc)₂, Pd(TFA)₂, Pd(CH₃CN)Cl₂.

5. Process according to any of Claims 1 to 4,
wherein the process comprises additional process step f) :
f) addition of an acid.

6. Process according to Claim 5,
wherein the acid is selected from: H₂SO₄, CH₃SO₃H, CF₃SO₃H, PTSA.

7. Process according to any of Claims 1 to 6,
wherein the MeOH / HCOOH ratio based on the employed volume is in the range from 1.5 : 0.5 to 1.2 : 0.8.

8. Process according to any of Claims 1 to 7,
wherein R¹, R², R³, R⁴ are each independently selected from: -(C₁-C₁₂)-alkyl, -O-(C₁-C₁₂)-alkyl, -(C₄-C₁₄)-aryl, - O-(C₄-C₁₄)-aryl, cycloalkyl, -(C₁-C₁₂)-heteroalkyl, -O-(C₁-C₁₂)-heteroalkyl, -(C₃-C₁₄)-heteroaryl, -O-(C₃-C₁₄)-heteroaryl, -COO-alkyl, -COO-aryl, -C-O-alkyl, -C-O-aryl, NH₂, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl.

9. Process according to any of Claims 1 to 8,
wherein R¹, R², R³, R⁴ are each independently selected from: -(C₁-C₁₂)-alkyl, -(C₄-C₁₄)-aryl, cycloalkyl, -(C₁-C₁₂) -heteroalkyl, -(C₃-C₁₄) -heteroaryl, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl.

10. Process according to any of Claims 1 to 9,
wherein R¹, R², R³, R⁴ are each independently selected from: -(C₁-C₁₂) -alkyl, cycloalkyl, -(C₃-C₁₄) -heteroaryl and the residues are also capable of forming a larger condensed ring;
wherein the recited alkyl groups, cycloalkyl, heteroaryl groups may be substituted as follows:
-(C₁-C₁₂)-alkyl, -O-(C₁-C₁₂)-alkyl, halogen,
and at least one of the radicals R¹, R², R³, R⁴ does not represent phenyl.

11. Process according to any of Claims 1 to 10,
wherein R¹, R⁴ are each independently selected from: - (C₁-C₁₂) -alkyl, cycloalkyl and the residues are also capable of forming a larger condensed ring;
wherein the recited alkyl groups, cycloalkyl may be substituted as follows:
-(C₁-C₁₂)-alkyl, -O-(C₁-C₁₂)-alkyl, halogen.

12. Process according to any of Claims 1 to 11,
wherein R², R³ each independently represent -(C₃-C₁₄) - heteroaryl,
wherein the recited heteroaryl groups may be substituted as follows:
-(C₁-C₁₂) -alkyl, -O-(C₁-C₁₂) -alkyl, halogen.

13. Process according to any of Claims 1 to 12,
wherein the compound of general formula (**I**) has the structure (**2**):

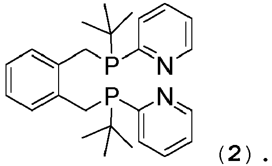

14. Process according to any of Claims 1 to 12,
wherein the compound of general formula (**I**) has the structure (**3**):

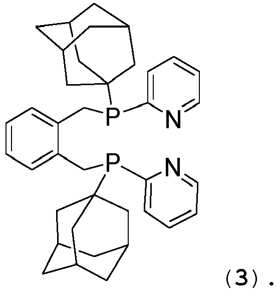

15. Compound having the structure (3):

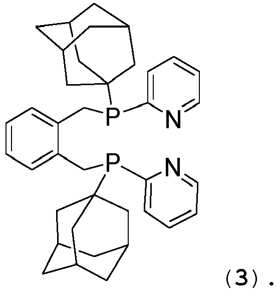

## Revendications

1. Procédé comprenant les étapes de procédé :
a) ajout d'une oléfine ;
b) ajout d'un composé, qui comprend du Pd, le Pd étant en état de former un complexe ;
c) ajout d'un composé de formule générale (I) : 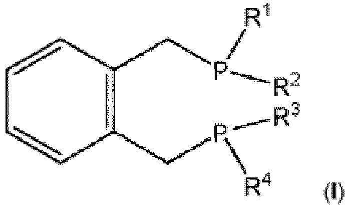
R¹, R², R³, R⁴ à chaque fois indépendamment les uns des autres étant choisis parmi : -H, -C₁-₁₂-alkyle, -O-C₁₋₁₂-alkyle, -C₄₋₁₄-aryle, -O-C₄-₁₄-aryle, cycloalkyle, -C₁₋₁₂-hétéroalkyle, -OC₁₋₁₂-hétéroalkyle, -C₃₋₁₄-hétéroaryle, -O-C₃₋₁₄-hétéroaryle, -COO-alkyle, -COO-aryle, -C-O-alkyle, -C-O-aryle, NH₂, halogène et les radicaux étant aussi aptes à la formation d'un cycle condensé plus grand ;
les groupes mentionnés alkyle, aryle, cycloalkyle, hétéroalkyle, hétéroaryle pouvant être substitués comme suit :
-C₁₋₁₂-alkyle, -O-C₁₋₁₂-alkyle, halogène ;
et au moins un des radicaux R¹, R², R³, R⁴ ne représentant pas phényle ;
d) ajout de MeOH et de HCOOH,
le rapport MeOH/HCOOH, par rapport au volume utilisé, se situant dans la plage de 1,55:0,45 à 1,1:0,9 ;
e) chauffage du mélange réactionnel, l'oléfine étant transformée en un ester méthylique.

2. Procédé selon la revendication 1,
aucun gaz de CO n'étant introduit dans le mélange réactionnel.

3. Procédé selon la revendication 1 ou 2,
HCOOH servant de seule source de CO pour la réaction.

4. Procédé selon l'une quelconque des revendications 1 à 3,
le composé dans l'étape de procédé b) étant choisi parmi :
Pd(acac)₂, PdCl₂, Pd(dba)₃*CH₃Cl (dba = dibenzylidèneacétone), Pd(OAc)₂, Pd(TFA)₂, Pd(CH₃CN)Cl₂.

5. Procédé selon l'une quelconque des revendications 1 à 4,
le procédé comprenant l'étape de procédé supplémentaire f) :
f) ajout d'un acide.

6. Procédé selon la revendication 5,
l'acide étant choisi parmi : H₂SO₄, CH₃SO₃H, CF₃SO₃H, PTSA.

7. Procédé selon l'une quelconque des revendications 1 à 6,
le rapport MeOH/HCOOH, par rapport au volume utilisé, se situant dans la plage de 1,5:0,5 à 1,2:0,8.

8. Procédé selon l'une quelconque des revendications 1 à 7,
R¹, R², R³, R⁴ à chaque fois indépendamment les uns des autres étant choisis parmi : -C₁₋₁₂-alkyle, -OC₁₋₁₂-alkyle, -C₄₋₁₄-aryle, -O-C₄₋₁₄-aryle, cycloalkyle, -C₁₋₁₂-hétéroalkyle, -O-C₁₋₁₂-hétéroalkyle, -C₃₋₁₄-hétéroaryle, -O-C₃₋₁₄-hétéroaryle, -COO-alkyle, -COO-aryle, -C-O-alkyle, -C-O-aryle, NH₂, halogène et les radicaux étant aussi aptes à la formation d'un cycle condensé plus grand ;
les groupes mentionnés alkyle, aryle, cycloalkyle, hétéroalkyle, hétéroaryle pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

9. Procédé selon l'une quelconque des revendications 1 à 8,
R¹, R², R³, R⁴ à chaque fois indépendamment les uns des autres étant choisis parmi : -C₁₋₁₂-alkyle, -C₄₋₁₄-aryle, cycloalkyle, -C₁₋₁₂-hétéroalkyle, -C₃₋₁₄-hétéroaryle, halogène et les radicaux étant aussi aptes à la formation d'un cycle condensé plus grand ;
les groupes mentionnés alkyle, aryle, cycloalkyle, hétéroalkyle, hétéroaryle pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

10. Procédé selon l'une quelconque des revendications 1 à 9,
R¹, R², R³, R⁴ à chaque fois indépendamment les uns des autres étant choisis parmi : -C₁₋₁₂-alkyle, cycloalkyle, -C₃₋₁₄-hétéroaryle et les radicaux étant aussi aptes à la formation d'un cycle condensé plus grand ;
les groupes mentionnés alkyle, cycloalkyle, hétéroaryle pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

11. Procédé selon l'une quelconque des revendications 1 à 10,
R¹, R⁴ à chaque fois indépendamment l'un de l'autre étant choisis parmi : -C₁₋₁₂-alkyle, cycloalkyle et les radicaux étant aussi aptes à la formation d'un cycle condensé plus grand ;
les groupes mentionnés alkyle, cycloalkyle pouvant être substitués comme suit :
-C₁₋₁₂-alkyle, -O-C₁₋₁₂-alkyle, halogène.

12. Procédé selon l'une quelconque des revendications 1 à 11,
R², R³ à chaque fois indépendamment l'un de l'autre représentant -C₃₋₁₄-hétéroaryle,
les groupes mentionnés hétéroaryle pouvant être substitués comme suit :
-C₁₋₁₂-alkyle, -O-C₁₋₁₂-alkyle, halogène.

13. Procédé selon l'une quelconque des revendications 1 à 12,
le composé de formule générale (I) présentant la structure (2) :

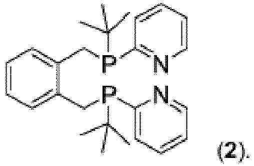

14. Procédé selon l'une quelconque des revendications 1 à 12,
le composé de formule générale (I) présentant la structure (3) :

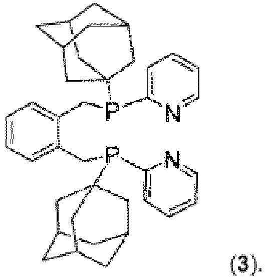

15. Composé, qui présente la structure (3) :
